# EUROPEAN PATENT APPLICATION

(11) **EP 3 913 169 A1**
(43) Date of publication of application: **24.11.2021**
(21) Application number: 21175537.6
(22) Date of filing: 24.05.2021
(51) Int. Cl.: E05B 1/00, E05B 17/10, E05B 43/00, E05B 17/22, A61L 2/10

(54) **GERMICIDAL HANDLES**

(30) Priority: 22.05.2020 US 202063028761 P
(71) Applicant: Simmonds Precision Products, Inc., Vergennes, VT 05491 (US)
(72) Inventor: CARINI, Peter, Underhill, 05489 (US)
(74) Representative: Dehns

(57) **Abstract**

A germicidal system includes a handle for opening a door or compartment. An ultra-violet illuminator is mounted proximate the handle for irradiating a surface of the handle to reduce pathogens from the handle between uses. The illuminator can include at least one LED that emits in ultra-violet wavelengths. A sensor can be included proximate the handle. A controller can be operatively connected to the sensor and to the illuminator. The controller can be configured to activate the illuminator based on detection of a user by the sensor. The controller can be configured to activate the illuminator for a predetermined amount of time for each use of the handle.

## Description

### BACKGROUND

### 1. Field

The present disclosure relates to sanitizing, and more particularly to sanitizing handles such as door and compartment handles in aircraft.

### 2. Description of Related Art

Air travel was greatly reduced due to the COVID-19 pandemic and subsequent mitigations. Improving sanitation and cleanliness in transport vehicles to ensure the healthy transport of passengers is a paramount consideration for the future of commercial transportation.

The conventional techniques have been considered satisfactory for their intended purpose. However, there is an ever present need for improved systems and methods for sanitizing aircraft interiors and the like. This disclosure provides a solution for this need.

### SUMMARY

A germicidal system includes a handle for opening a door or compartment. An ultra-violet illuminator is mounted proximate the handle for irradiating a surface of the handle to reduce pathogens from the handle between uses.

The handle can include a knob connected to a door mount. The illuminator can be mounted in the door mount and can be directed toward the knob. The illuminator can include a plurality of LEDs arranged in a ring in the door mount, encircling a neck of the knob.

The illuminator can include at least one LED that emits in ultra-violet wavelengths. The illuminator can emit modulated pulses in UV-C. The illuminator can be within 6 inches (15.24 cm) of the surface.

A sensor can be included proximate the handle. A controller can be operatively connected to the sensor and to the illuminator. The controller can be configured to activate the illuminator based on detection of a user by the sensor. The controller can be configured to activate the illuminator for a predetermined amount of time for each use of the handle. The controller can be configured to deactivate the illuminator after the predetermined amount of time. The predetermined amount of time can be between 2 seconds and 20 seconds, inclusive of endpoints. The controller can be configured to interrupt illumination from the illuminator if needed before conclusion of the predetermined amount of time. The controller can be configured to delay after detection of a user before activating the illuminator.

A battery for power storage can be operatively connected to the controller and to the illuminator to supply power to the illuminator and controller. An energy harvester can be operatively connected to the battery for recharging the battery.

A visible indicator can be operatively connected to the controller. The controller can be configured to activate the visible indicator while the illuminator is activated to signal to a user that the illuminator is active.

The handle can include a knob wherein the illuminator is mounted in the knob. The knob can have optical properties to transmit ultra-violet illumination to an outer surface of the knob.

The handle can include a latch mounted to a compartment mount. The compartment mount can include a finger recess to allow manual access to a back side of the latch. The illuminator can be mounted in the recess directed toward the latch. The illuminator can include an array of LEDs mounted in the finger recess.

The handle can includes a grip member mounted to and spaced apart from a door mount. The illuminator can be mounted in the door mount directed toward the grip member. The illuminator can include an array of LEDs mounted in the door mount.

These and other features of the systems and methods of the subject disclosure will become more readily apparent to those skilled in the art from the following detailed description of the preferred embodiments taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, preferred embodiments thereof will be described in detail herein below with reference to certain figures, wherein:
Fig. 1 is a schematic perspective view of an embodiment of a system constructed in accordance with the present disclosure, showing a knob with an ultra-violet illuminator;
Fig. 2 is a schematic view of another embodiment of a system, showing a knob with an ultra-violet illuminator inside;
Fig. 3 is a schematic view of another embodiment of a system, showing a latch with an ultra-violet illuminator; and
Fig. 4 is a schematic view of another embodiment of a system, showing a grip member with an ultra-violet illuminator mounted in an adjacent door mount.

### DETAILED DESCRIPTION OF THE PREFERRED EMBODIMENTS

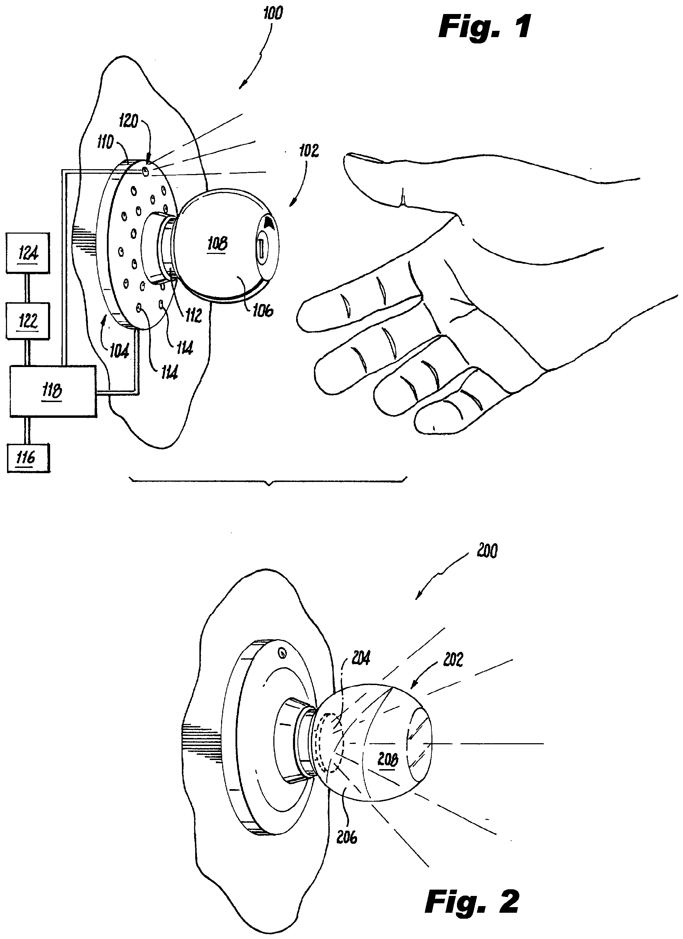

Reference will now be made to the drawings wherein like reference numerals identify similar structural features or aspects of the subject disclosure. For purposes of explanation and illustration, and not limitation, a partial view of an embodiment of a system in accordance with the disclosure is shown in Fig. 1 and is designated generally by reference character 100. Other embodiments of systems in accordance with the disclosure, or aspects thereof, are provided in Figs. 2-4, as will be described. The systems and methods described herein can be used for reduction or elimination of antigens from surfaces of door handles, compartment handles, and the like, such as for use in aircraft.

The germicidal system 100 includes a handle 102 for opening a door or compartment. An ultra-violet illuminator 104 is mounted proximate the handle 102 for irradiating a surface 106 of the handle 102 to reduce pathogens from the handle 102 between uses. The handle 102 includes a knob 108 connected to a door mount 110 by a neck 112. The illuminator 104 is mounted in the door mount 110 and is directed to shine illumination toward the knob 108. The illuminator 104 includes a plurality of light emitting diodes (LEDs) 114 (not all of which are numbered in Fig. 1 for sake of clarity) arranged in a ring in the door mount 110, encircling the neck 112 of the knob 108. The LEDs 114 emits in ultra-violet wavelengths, which can be pulses of UV-C illumination. Pulsing or modulating the illuminator 104 can save power while maintaining effectiveness. The illuminator 104 is within 6 inches (15.24 cm) of the surface, which in the UV-C wavelengths can reduce and/or eliminate viral antigens, bacterial antigens, fungal antigens, and the like from the surface 106 of the handle 102.

A sensor 116 is included proximate the handle 102, which means in or nearby the handle 102, e.g. co-located with the LEDs 114 or any suitable location. A controller 118 is operatively connected to the sensor 116 and to the illuminator 104. The controller 118 is configured to activate the illuminator 104 based on detection of a user by the sensor 116. For example, if the sensor 116 is a proximity sensor, the controller 118 can activate the illuminator 104 when the sensor 116 detects proximity of a person. Or if the sensor 116 is a touch sensor, the controller 118 can activate the illuminator 104 when a user touches the sensor 116, which can be, e.g. in the surface 106 of the handle 102. Any other suitable type of sensor can be used.

The controller 118 is configured to activate the illuminator 104 for a predetermined amount of time for each use of the handle 102, and to deactivate the illuminator 104 after the predetermined amount of time, which can be between 2 seconds to 20 seconds or 10 seconds and 20 seconds, inclusive of endpoints, for example. The controller 118 can be is configured to interrupt illumination from the illuminator 104 if needed before conclusion of the predetermined amount of time. For example, if a user touches the handle 102 while the illuminator 104 is active before the predetermined amount of time expires. The controller 118 can be configured to delay after detection of a user before activating the illuminator 104, e.g. to give the user time to use the handle 102 before activating the illuminator 104.

Since ultra-violet rays are not visible to humans, a visible indicator 120, such as a visible light or other indicia, can be operatively connected to the controller 118. The visible indicator 120 can be co-located with the LEDs 114, or in any other suitable location. It can serve as an indicator/warning highlighting the decontaminated area. The controller 118 can be configured to activate the visible indicator 120 while the illuminator 104 is activated to signal to a user that the illuminator 104 is active.

The system 100 can be connected to wired power, however it is also contemplated that a battery 122 for power storage can be operatively connected to the controller 118 and to the illuminator 104 to supply power to the illuminator 104 and controller 118. If it is not desirable to replace the battery 122, an energy harvester 124 can be optionally be connected to the battery 122 for recharging the battery 122. Any suitable type of energy harvester can be used, such as inertial, solar, thermal, or the like.

With reference now to Figs. 2-4, other configurations of systems 200, 300, 400 are disclosed, which can utilize the same type of sensor 116, controller 118, indicator 120, battery 122, and/or energy harvester 124 as described above. As shown in Fig. 2, the handle 202 can include a knob 208 wherein the illuminator 204, e.g. a single large UV-C LED (or array of UV-C LEDs), is mounted in the knob 208. The knob 208 has optical properties, e.g., transparency, to transmit ultra-violet illumination to an outer surface 206 of the knob 208.

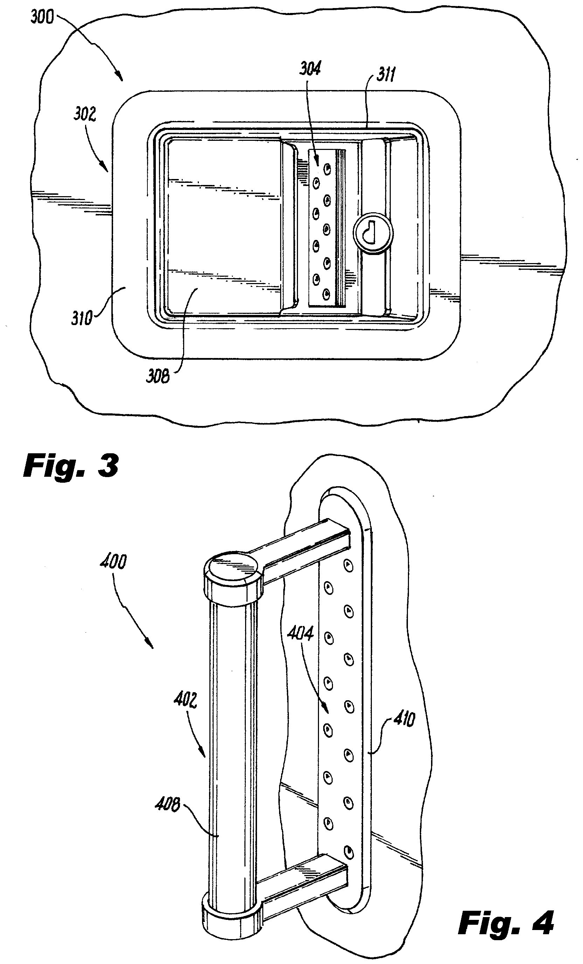

With reference now to Fig. 3, the handle 302 includes a latch 308, which can be transparent to transmit UV-C, mounted to a compartment mount 310, which can be mounted to an overhead bin, galley compartment, or the like. The compartment mount 310 includes a finger recess 311 to allow manual access to a back side of the latch 308. The illuminator 304, e.g. an array of UV-C LEDs (not individually numbered in Fig. 3 for sake of clarity), is mounted in the recess 311 directed toward the latch 308.

With reference now to Fig. 4, the handle 402 includes a grip member 408 mounted to and spaced apart from a door mount 410. The grip member 408 does not move relative to the door mount 410. The illuminator 404 is mounted in the door mount 410 and is directed toward the grip member 408. The illuminator 404 includes an array of UV-C LEDs (not individually numbered for sake of clarity) mounted in the door mount 410.

While four different types of handles are disclosed herein, those skilled in the art will readily appreciate that any other suitable type of handles, door knobs, latches, buttons, or the like can be used in systems as disclosed herein. Potential benefits of systems and methods as disclosed herein include automated rapid disinfection of frequently used surfaces immediately after human contact.

The methods and systems of the present disclosure, as described above and shown in the drawings, provide for reduction or elimination of antigens from surfaces of door handles, compartment handles, and the like, such as for use in aircraft. While the apparatus and methods of the subject disclosure have been shown and described with reference to preferred embodiments, those skilled in the art will readily appreciate that changes and/or modifications may be made thereto without departing from the scope of the subject disclosure.

## Claims

1. A germicidal system comprising:
a handle for opening a door or compartment; and
an ultra-violet illuminator mounted proximate the handle for irradiating a surface of the handle to reduce pathogens from the handle between uses.

2. The system as recited in claim 1, wherein the handle includes a knob connected to a door mount, and wherein the illuminator is mounted in the door mount and is directed toward the knob; optionally wherein the illuminator includes a plurality of LEDs arranged in a ring in the door mount, encircling a neck of the knob.

3. The system as recited in claim 1, wherein the handle includes a knob wherein the illuminator is mounted in the knob, and wherein the knob has optical properties to transmit ultra-violet illumination to an outer surface of the knob.

4. The system as recited in claim 1, wherein the handle includes a latch mounted to a compartment mount, wherein the compartment mount includes a finger recess to allow manual access to a back side of the latch, and wherein the illuminator is mounted in the recess directed toward the latch; optionally wherein the illuminator includes an array of LEDs mounted in the finger recess.

5. The system as recited in claim 1, wherein the handle includes a grip member mounted to and spaced apart from a door mount, wherein the illuminator is mounted in the door mount directed toward the grip member; optionally wherein the illuminator includes an array of LEDs mounted in the door mount.

6. The system as recited in any preceding claim, wherein the illuminator includes at least one LED that emits in ultra-violet wavelengths.

7. The system as recited in claim 6, wherein the illuminator emits modulated pulses in UV-C.

8. The system as recited in any preceding claim, further comprising:
a sensor proximate the handle; and
a controller operatively connected to the sensor and to the illuminator, wherein the controller is configured to activate the illuminator based on detection of a user by the sensor.

9. The system as recited in claim 8, wherein the controller is configured to activate the illuminator for a predetermined amount of time for each use of the handle and to deactivate the illuminator after the predetermined amount of time.

10. The system as recited in claim 9, wherein the predetermined amount of time is between 2 seconds and 20 seconds, inclusive of endpoints; and/or
wherein the controller is configured to interrupt illumination from the illuminator if needed before conclusion of the predetermined amount of time.

11. The system as recited in claim 9 or 10, wherein the controller is configured to delay after detection of a user before activating the illuminator.

12. The system as recited in claim 9, 10 or 11, further comprising a battery for power storage operatively connected to the controller and to the illuminator to supply power to the illuminator and controller.

13. The system as recited in claim 12, further comprising an energy harvester operatively connected to the battery for recharging the battery.

14. The system as recited in any one of claims 8-13, further comprising a visible indicator operatively connected to the controller, wherein the controller is configured to activate the visible indicator while the illuminator is activated to signal to a user that the illuminator is active.

15. The system as recited in any preceding claim, wherein the illuminator is within 6 inches (15.24 cm) of the surface.
